# EUROPEAN PATENT SPECIFICATION

(11) **EP 1 277 510 B1**
(45) Date of publication and mention of the grant of the patent: **21.09.2005**
(21) Application number: 02015454.8
(22) Date of filing: 11.07.2002
(51) Int. Cl.: B01D 63/02, A61M 1/18

(54) **Dialyzer**
Dialysator
Dialyseur

(30) Priority: 17.07.2001 US 907073; 01.10.2001 US 969123
(43) Date of publication of application: 22.01.2003
(73) Proprietor: Fresenius USA, Inc., Lexington, MA 02173 (US)
(72) Inventor: Lee Jacobsen, Veronica, Sandy Utah (US); Kuykendall, Stephen, North Ogden Utah (US); Tuominen, Olli, Marlboro MA 01752 (US); McGhee, Troy, North Ogden Utah (US)
(74) Representative: Laufhütte, Dieter

(56) References cited:
- FR-A- 2 542 203
- US-A- 4 201 673
- US-A- 4 219 426
- US-A- 6 074 559
- PATENT ABSTRACTS OF JAPAN vol. 1997, no. 02, 28 February 1997 (1997-02-28) -& JP 08 280796 A (CYTEC KK), 29 October 1996 (1996-10-29) -& DATABASE WPI Derwent Publications Ltd., London, GB; AN 1997-015202 XP002258101 & JP 08 280796 A

## Description

### Field of the Invention

This invention relates to the field of dialyzers typically used in hemodialysis and related medical procedures, and in particular to a design having a lengthened useful life, together with a method for manufacturing the same.

### Background of the Invention

Patients with kidney disease suffer from the adverse effects of toxin build-up in their blood. Dialysis is a process which employs an artificial kidney to remove those toxins. In hemodialysis a dialyzer is used which contains a semi-permeable membrane dividing the dialyzer into two chambers. Blood is pumped through one chamber and a dialysis solution through the second. As the blood flows by the dialysis fluid, separated by the semipermeable membrane, blood impurities such as urea and creatinine diffuse through the semi-permeable membrane into the dialysis solution by the diffusion, convection and absorption. The electrolyte concentration of the dialysis fluid is set so as to maintain electrolytic balance within the patient.

Dialyzers often use a large number of microfibers encased in a chamber. The chamber is often a hollow cylinder open at both ends. Thousands of hollow semipermeable microfibers carry blood from one end to the opposite end so that blood flows through the microfibers in a first direction. Dialysate ports are also present on opposite ends of the chamber. One port carries dialysate into the chamber, the dialysate flows through the chamber in a countercurrent direction to the blood flow, and the other port carries the dialysate out of the chamber. The solute removal thus takes place across the semipermeable membrane that is the microfiber wall. This design produces a high surface area for solute removal in a relatively low volume device.

One significant challenge is to connect the microfiber interior channels to the blood lines, so that blood flows smoothly from the arterial blood line, into the microfiber interior channels where it can pass into and through the dialyzer chamber, and out the other end of the microfiber interior channels to the venous blood line.

This is done by filling the open-ended cylinder-shaped dialyzer chamber with microfibers extending in the longitudinal direction. Each end of the cylinder is threaded to receive a cap closing the end. The dialyzer is then positioned in a centrifuge to allow rotation about an axis perpendicular to the central longitudinal axis, wherein the axis of rotation extending through the midpoint of the cylinder. A liquid potting material such as epoxy or urethane is then injected into the dialysate ports on each end of the chamber, and the dialyzer is spun in the centrifuge. The centripetal force produced by the rotation in the centrifuge forces the potting material to each end, where it sets and hardens.

The dialysate ports in the cylinder wall near each end of the dialyzer present special challenges. It is desirable for the microfibers to be supported by the cylinder wall throughout their length, or as much of their length as possible, so that the dialysate fluid flow pressure on the microfibers does not distort them. Such distortion can result in their breaking, which allows mingling of blood and dialysate, or can obstruct the flow of blood through the microfiber interior channels.

Support for the microfibers is achieved in some designs by separating the dialysate ports from the dialyzer chambers with a dialysate fluid distribution ring. In the dialyzer disclosed in JP 8 280 796 A additional dialysate fluid guide rings are used to obtain a uniform flow of the dialysate fluid. The dialysate fluid distribution ring is effectively an extension of the dialyzer cylinder wall. The dialysate port accesses the dialyzer chamber by means of an intermediate space defined at the radial inner side by the dialysate fluid distribution ring and at the radially outer side by an outer wall. The dialysate fluid distribution ring thus extends lateral support to the microfibers around the circumference of the microfiber bundle while still enhancing dialysate fluid distribution between the dialysate ports and the chamber.

In practice, this design is quite effective. It has been discovered, however, that the microfibers tend to fail first at a particular location due apparently to an artifact of the potting process. As the dialyzer is spun in a centrifuge to force potting material to each end to encase the microfibers, the surface closest to the axis of spinning rotation assumes a curve. It is believed that this is because the centripetal force exerted on the potting material is in proportion to the distance of the potting material from the axis of rotation, much as the outward force on a merry-go-round is greater at the edge than at the center. In order to maintain constant centripetal force along the potting material surface during the centrifuge operation, that surface assumes a curvature that maintains a constant distance from the axis of centrifuge rotation. That resulting curvature departs slightly from the plane resulting from cutting off the ends of the potting material to expose the microfiber interior channels.

Like the cut-off end of the potting material, the dialysate fluid distribution ring edge has in the past been planar. Because the dialysate fluid distribution ring edge has been planar while the potting material surface has been curved, the width of the gap between the dialysate fluid distribution ring edge and the potting material has varied. This gap represents the space in which the microfiber bundle is unsupported by the dialyzer cylinder wall or by the dialysate fluid distribution ring. The dialysate fluid flow pressure between the microfiber interior channel and the dialyzer chamber interior tends to flex or distort the microfibers. Because pressure produces a force in proportion to the area on which it acts, the force produced at areas of relatively large gap between the dialysate fluid distribution ring edge and the potting material surface is different from the force produced at areas of relatively small gap between the dialysate fluid distribution ring edge and the potting material surface. It has been found that this differential force is responsible for much of the microfiber distortion, rupturing and obstruction seen in the initial stages of dialyzer destruction.

FR 2 542 203 A discloses a dialyzer wherein the microfibres are retained by tubular elements which are embedded in the potting materials. Said tubular elements have dialysate distribution openings which are arranged along the line of intersection between said tubular retaining elements and the cylindrical surface of the potting materials, such that the dialysate fluid flows over the full length of the microfibres and dead spaces are avoided.

It is desirable for dialyzers to be filled with microfibers in a manner that supports the dialysate fluid flow pressure on the microfiber bundle in a uniform way to avoid differential forces on the microfibers which distorts, ruptures or obstructs them.

### Summary of the Invention

The present invention relates to a dialyzer as claimed in claim 1.

The present invention overcomes the deficiencies of existing systems by use of a novel design for the dialyzer dialysate fluid distribution ring. This dialysate fluid distribution ring separates the dialyzer interior chamber from the dialysate ports. The dialysate fluid distribution ring edge distal from the centrifuge axis of rotation assumes a shape defined as an annulus cut by a cylinder perpendicular to the axis of the annulus.

### Detailed Description of a Preferred Embodiment

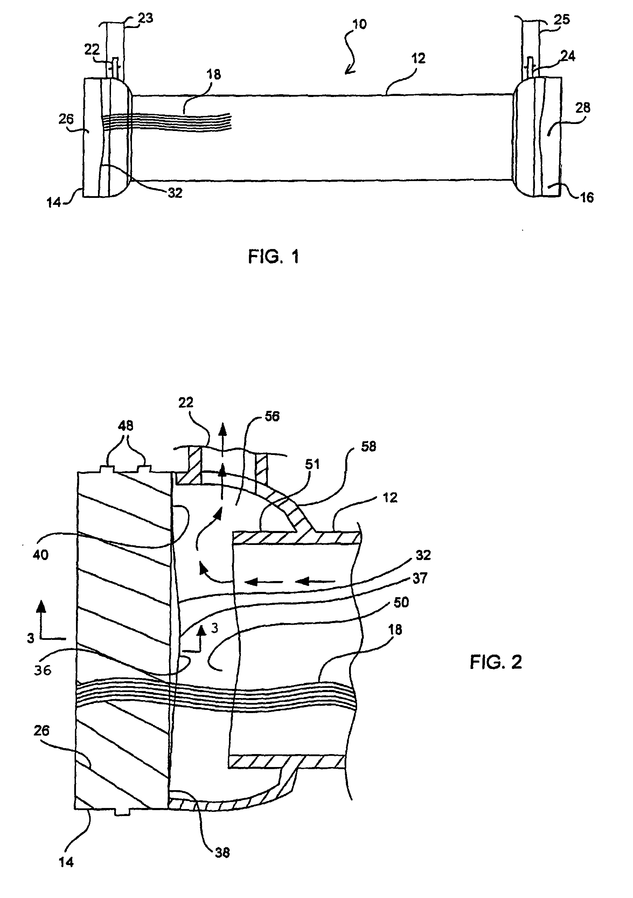

A dialyzer 10 in accordance with a preferred embodiment of the invention is shown in Fig. 1. The overall shape of the dialyzer 10 is cylindrical, comprising a cylindrical body 12, a blood line inlet end 14 and a blood line outlet end 16.

The ends 14 and 16 are preferably enlarged compared to the chamber body to accommodate a distribution ring described below. Proximate to the blood inlet end 14 is a dialysate outlet port 22, and proximate to the blood outlet end 16 is a dialysate inlet port 24. The cylindrical body 12 is filled with a microfiber bundle 18, only a fragment of which is shown in Fig. 1 for purposes of clarity. The microfibers 18 are potted in a polyurethane potting material 26 and 28 at each end 14 and 16. The dialysate inlet port 24 connects to a dialysate inlet line 25, and the dialysate outlet port 22 connects to a dialysate outlet line 23.

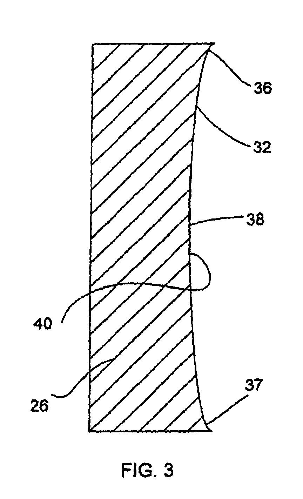

An enlarged sectional view of one end 14 of the dialyzer 10 is shown in Fig. 2 and Fig. 3. Fig. 3 omits all but the potting material for clarity. A portion of the microfiber bundle 18 is shown in Fig. 2, embedded in the potting material 26, while the rest of the microfiber bundle 18 is omitted for clarity. As the figures show, the interior surface 32 of the potting material 26 is not planar. Rather, geometrically speaking, it is the surface defined by the intersection of a cylinder with another cylinder. In this case, the first cylinder is the potting material 26. The intersecting, or cutting, cylinder which defines the potting material surface is an imaginary cylinder with a longitudinal axis extending up and down and intersecting with the longitudinal coaxial axes of the potting material 26 cylinder and the dialyzer cylindrical body 12. The resultant interior surface 32 of the potting material 26 has a circumferential edge that undulates. The overall thickness of the potting material 26 thus varies from relatively thick at two opposite points 36 and a point 37 opposite 36, to relatively thin at two opposite points that are offset 90° from point 36, at points 38 and 40.

The potting material 26 ensures that the blood is confined to the interior channels of the microfiber bundle, and is unable to leak into the dialyzer chamber. A fitting (not shown) threaded onto the threads 48 of the dialyzer ends 14 and 16 serve to connect the blood lines to the dialyzer ends 14 and 16. Blood thereby flows from the arterial blood line into the interior bundle of the microfibers of the microfiber bundle 18, through the microfiber interior channels from one end 14 to the other end 16 of the dialyzer, and into the venous blood line (not shown) via another fitting (not shown) connecting that end 16 to the venous blood line.

A set of threads 48 is molded into the dialyzer 10 ends 14 and 16 to allow the ends to be capped during the step of centrifuging the potting material into the dialyzer chamber, and also to allow attachment to a fitting for connection to the blood lines. This portion of the manufacturing process is known in the art, and is not further described here.

The design and configuration of the dialysate fluid distribution ring 51 is evident from Fig. 2. The dialysate fluid distribution ring 51 is an annular extension of the cylindrical dialyzer body 12. The dialyzer end 14 is radially spaced away from the dialysate fluid distribution ring 51 to define an annular space 56 between the end housing 58 and the dialysate fluid distribution ring 51.

In operation, blood flows from an arterial blood line (not shown) and into the interior channels of the microfibers comprising the microfiber bundle 18. At the same time, dialysate flows in the countercurrent direction to the blood flow. Dialysate flows from the inlet dialysate line 24 and through the inlet port 24 to enter the interior chamber of the dialyzer 10. The dialysate flows through the dialyzer interior chamber on the outside of the microfibers. The blood is thus separated from the dialysate by the semi-permeable membranes of the microfiber walls, which allow the transfer of liquids, toxins and nutrients by the diffusion, convection, & absorption. The dialysate then exits the dialyzer 10 through the dialysate outlet port 22 and into the dialysate outlet line 23.

The dialysate fluid distribution ring circumferential edge 50 in past designs has been planar. Because the potting material 26 surface is not planar, due to the effect of the centrifugal molding process, the result in past designs has been a varying gap between the potting material surface and the distribution ring circumferential edge. Because the dialysate fluid distribution ring 51 provides support to the microfiber bundle 18 against the dialysate fluid flow pressure exerted during dialysis procedures, this support varies along the circumference of the dialysate fluid distribution ring 51 edge in past designs. This varying support tends to distort, rupture or obstruct the microfibers.

As show in Fig. 2, the design of the dialysate fluid distribution ring 51 circumferential edge in the present invention is not planar. It instead is of an undulating shape that conforms to the undulating shape of the potting material 26 surface. This results in a gap between the potting material 26 surface and the dialysate fluid distribution ring 51 circumferential edge that does not vary but is instead substantially constant.

This substantially constant gap allows for substantially constant support of the microfiber bundle 18. "Substantially constant gap" means for purposes of the claims below a gap which may be, but is not necessarily, exactly uniform, but which varies by an amount that lessens the distortion, rupturing or obstruction of the microfibers in comparison to the prior art planar circumferential edge of the dialysate fluid distribution ring.

Said gap or distance a preferably between 0.32 and 0.95 cm (one - eighth and three-eighths inch), and more preferably between 0.51 and 0.64 cm (one-fifth and one-fourth inch).

Results of experimental tests show that the non-planar distribution ring of the present invention does indeed lessen the failure rate of the dialyzer. Three types of dialyzers were tested: prototypes having the novel distribution ring design described herein; production samples of Model F80A of Fresenius Medical Care; and Model CA210 of Baxter Laboratories.

The pressure used in this testing was 3.45 bar (50 psi) and the flow rate was 15.16 l/min (4 gpm) per dialyzer. The testing included several phases for each cycle: phase 1 included 3 seconds of flow through the dialysate port only; phase 2 included 3 seconds of flow through the blood port only, phase 3 included 3 seconds of flow through the dialysate and blood ports, and phase 4 was 3 seconds with no flow. Each "cycle was defined as 11 minutes of sequential and repetitious 4-phase testing.

The results are presented in the table below, which shows the number of dialysis cycles completed before each failure for each sample, the average number of such cycles for each of the three designs, and the Standard Deviation. At the end of each cycle of testing, the dialyzers were tested for a bubble point failure to detect the fiber shear. If the dialyzer failed the bubble point test, then it was categorized as failed due to a fiber shear.

| **Sample Type: F80A** | **Failed Cycle** | **Sample Type: Prototype** | **Failed Cycle** | **Sample Type: Baxter CA 210** | **Failed Cycle** |
|---|---|---|---|---|---|
| Sample 1 | 1 | Sample 1 | 26 | Sample 1 | 1 |
| Sample 2 | 4 | Sample 2 | 14 | Sample 2 | 1 |
| Sample 3 | 1 | Sample 3 | 14 | Sample 3 | 1 |
| Sample 4 | 1 | Sample 4 | 12 | Sample 4 | 1 |
| Sample 5 | 1 | Sample 5 | 7 | Sample 5 | 1 |
| Sample 6 | 1 | Sample 6 | 25 | Sample 6 | 1 |
| Sample 7 | 1 | Sample 7 | 7 | Sample 7 | 1 |
| Sample 8 | 1 | Sample 8 | 26 | Sample 8 | 1 |
| Sample 9 | 6 | Sample 9 | 12 | Sample 9 | 1 |
| Sample 10 | 1 | Sample 10 | 8 | Sample 10 | 1 |
| **Average** | **1.8 cycles** | **Average** | **15.1 cycles** | **Average** | **1 cycle** |
| **S.D.** | **1.75** | **S.D.** | **7.74** | **S.D.** | **0** |

It can be seen from these results that the design with a novel distribution ring in accordance with the present invention showed a much lower failure rate, or much higher number of cycles before failure. While these tests do not purport to duplicate exactly any actual dialysis procedure, it is believed that they fairly predict relative failure rates in such procedures.

## Claims

1. A dialyzer (10), comprising: a cylindrical body (12) having a first end (16) and a second end (14), the body (12) having an interior with a set of semi-permeable microfibers (18) extending from the first end (16) to the second end (14); a dialysate inlet port (24) proximate the first end (16) and a dialysate outlet port (22) proximate the second end (14); wherein the microfibers are encased in a first potting material (28) at the first end (16) and a second potting material (26) at the second end (14), wherein the potting materials (26, 28) surround and encase the microfibers in the body (12), and the potting materials (26, 28) each have an interior surface (32) with an undulating circumferential edge; a first annular shaped dialysate fluid distribution ring (51) proximate the first end (16) and a second annular shaped dialysate fluid distribution ring (51) proximate the second end (14), the dialysate fluid distribution rings (51) dividing the body interior from the ports (22, 24), wherein the dialysate fluid distribution rings (51) each have an undulating circumferential edge (50) that are a substantially constant distance from the undulating circumferential edges of the potting material (26, 28).

2. The dialyzer of claim 1, wherein the undulating circumferential edges of the potting materials (26, 28) and the dialysate fluid distribution rings (51) are geometrically defined by a cylinder intersecting another cylinder.

3. The dialyzer of claim 1, wherein the undulating circumferential edge of the potting materials (26, 28) includes a first pair of two opposite edges extending toward the body middle in relation to a second pair of two opposite edges at approximately 90° from the first pair of opposite edges.

4. The dialyzer of claim 1, wherein said distance is between 0.32 and 0.95 cm (one-eighth and three-eighths inch).

5. The dialyzer of claim 1, wherein said distance is between 0.51 and 0.64 cm (one-fifth and one-fourth inch).

## Patentansprüche

1. Dialysator (10), welcher umfasst: einen zylinderförmigen Körper (12) mit einem ersten Ende (16) und einem zweiten Ende (14), wobei der Körper (12) einen Innenraum mit einer Gruppe von sich von dem ersten Ende (16) zu dem zweiten Ende (14) erstreckenden semipermeablen Mikrofasem (18) aufweist; eine Dialysateinlassöffnung (24) nahe dem ersten Ende (16) und eine Dialysatauslassöffnung (22) nahe dem zweiten Ende (14); wobei die Mikrofasem am ersten Ende (16) in einer ersten Vergussmasse (28) und am zweiten Ende (14) in einer zweiten Vergussmasse (26) eingehüllt sind, wobei die Vergussmassen (26, 28) die Mikrofasern in dem Körper (12) umgeben und einhüllen und die Vergussmassen (26, 28) jeweils eine Innenfläche (32) mit einer wellenförmigen umlaufenden Kante aufweisen; einen ersten ringförmigen Dialysatfluid-Verteilungsring (51) nahe dem ersten Ende (16) und einen zweiten ringförmigen Dialysatfluid-Verteilungsring (51) nahe dem zweiten Ende (14), wobei die Dialysatfluid-Verteilungsringe (51) den Körperinnenraum von den Öffnungen (22, 24) unterteilen, wobei die Dialysatfluid-Verteilungsringe (51) jeweils eine wellenförmige umlaufende Kante (50) aufweisen, die einen im Wesentlichen konstanten Abstand von den wellenförmigen umlaufenden Kanten der Vergussmassen (26, 28) aufweisen.

2. Dialysator nach Anspruch 1, **dadurch gekennzeichnet, dass** die wellenförmigen umlaufenden Kanten der Vergussmassen (26, 28) und die Dialysatfluid-Verteilungsringe (51) durch einen einen anderen Zylinder schneidenden Zylinder geometrisch festgelegt werden.

3. Dialysator nach Anspruch 1, **dadurch gekennzeichnet, dass** die wellenförmige umlaufende Kante der Vergussmassen (26, 28) ein erstes Paar von zwei gegenüberliegenden Kanten umfasst, welche im Vergleich zu einem zweiten Paar von zwei gegenüberliegenden Kanten bei etwa 90° zum ersten Paar gegenüberliegender Kanten hin zur Körpermitte verlaufen.

4. Dialysator nach Anspruch 1, **dadurch gekennzeichnet, dass** der Abstand bei 0,32 bis 0,95 cm (1/8 bis 3/8 Zoll) liegt.

5. Dialysator nach Anspruch 1, **dadurch gekennzeichnet, dass** der Abstand bei 0,51 bis 0,64 cm (1/5 bis ¼ Zoll) liegt.

## Revendications

1. Dialyseur (10) comprenant : un corps cylindrique (12) ayant une première extrémité (16) et une seconde extrémité (14), le corps (12) ayant un intérieur avec un ensemble de microfibres semi-perméables (18) s'étendant de la première extrémité (16) à la seconde extrémité (14) ; un orifice d'entrée de dialysat (24) à proximité de la première extrémité (16) et un orifice de sortie de dialysat (22) à proximité de la seconde extrémité (14) ; où les microfibres sont renfermées dans un premier matériau d'empotage (28) à la première extrémité (16) et un second matériau d'empotage (26) à la seconde extrémité (14), où les matériaux d'empotage (26, 28) entourent et renferment les microfibres dans le corps (12), et les matériaux d'empotage (26, 28) ont chacun une surface intérieure (32) avec un bord circonférentiel ondulant ; un premier anneau de distribution de bain de dialyse de forme annulaire (51) proche de la première extrémité (16) et un second anneau de distribution de bain de dialyse de forme annulaire (51) proche de la seconde extrémité (14), les anneaux de distribution de bain de dialyse (51) séparant l'intérieur du corps des orifices (22, 24), où les anneaux de distribution de bain de dialyse (51) ont chacun un bord circonférentiel ondulant (50) qui sont à une distance sensiblement constante des bords circonférentiels ondulants du matériau d'empotage (26, 28).

2. Dialyseur selon la revendication 1, où les bords circonférentiels ondulants des matériaux d'empotage (26, 28) et les anneaux de distribution de bain de dialyse (51) sont définis de manière géométrique par un cylindre se croisant avec un autre cylindre.

3. Dialyseur selon la revendication 1, où le bord circonférentiel ondulant des matériaux d'empotage (26, 28) comprend une première paire de deux bords opposés s'étendant vers le milieu du corps relativement à une seconde paire de deux bords opposés selon environ 90° de la première paire de bords opposés.

4. Dialyseur selon la revendication 1, où ladite distance est entre 0,32 et 0,95 cm (un-huitième et trois-huitièmes de pouce).

5. Dialyseur selon la revendication 1, où ladite distance est entre 0,51 et 0,64 cm (un-cinquième et un-quatrième de pouce).
